(19) **Europäisches Patentamt European Patent Office Office européen des brevets**

(11) **EP 3 359 039 B1**

(12) **EUROPEAN PATENT SPECIFICATION**

(45) Date of publication and mention of the grant of the patent:
**14.07.2021 Bulletin 2021/28**

(21) Application number: **16853994.8**

(22) Date of filing: **07.10.2016**

(51) Int Cl.:
**A61B 5/145** (2006.01)  **G16H 50/20** (2018.01)
**G16H 20/10** (2018.01)  **A61B 5/00** (2006.01)
**A61K 38/28** (2006.01)  **A61M 5/168** (2006.01)
**G16H 10/40** (2018.01)  **G16H 20/17** (2018.01)

(86) International application number:
**PCT/SE2016/050966**

(87) International publication number:
**WO 2017/061943 (13.04.2017 Gazette 2017/15)**

(54) **MEDICAL ARRANGEMENTS AND A METHOD FOR DETERMINING PARAMETERS RELATED TO INSULIN THERAPY, PREDICTING GLUCOSE VALUES AND FOR PROVIDING INSULIN DOSING RECOMMENDATIONS**

MEDIZINISCHE ANORDNUNGEN UND VERFAHREN ZUR BESTIMMUNG VON PARAMETERN BEZÜGLICH EINER INSULINTHERAPIE, VORHERSAGE VON GLUCOSEWERTEN UND ZUR BEREITSTELLUNG VON INSULINDOSIERUNGSEMPFEHLUNGEN

SYSTÈMES MÉDICAUX ET PROCÉDÉ POUR DÉTERMINER DES PARAMÈTRES LIÉS À L'INSULINOTHÉRAPIE, POUR PRÉDIRE DES VALEURS DE GLUCOSÉ ET POUR FOURNIR DES RECOMMANDATIONS DE DOSAGE D'INSULINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2015 SE 1530154**

(43) Date of publication of application:
**15.08.2018 Bulletin 2018/33**

(73) Proprietor: **Dianovator AB**
**216 14 Limhamn (SE)**

(72) Inventor: **STÅHL, Fredrik**
**Limhamn 216 14 (SE)**

(74) Representative: **AWA Sweden AB**
**P.O. Box 5117**
**200 71 Malmö (SE)**

(56) References cited:
| | |
|---|---|
| WO-A1-2014/202233 | US-A1- 2005 272 640 |
| US-A1- 2009 006 061 | US-A1- 2009 253 614 |
| US-A1- 2010 125 241 | US-A1- 2010 198 020 |
| US-A1- 2010 262 117 | US-A1- 2011 047 108 |
| US-A1- 2012 109 687 | US-A1- 2012 123 234 |
| US-A1- 2012 246 106 | US-A1- 2014 052 095 |

US-A1- 2014 066 884    US-A1- 2015 164 414
US-B2- 8 818 782

- **CAMPETELLI ET AL.: 'Extended adaptive predictive controller with robust filter to enhance blood glucose regulation in type I diabetic subjects' COMPUTERS AND CHEMICAL ENGINEERING vol. 59, pages 243 - 251, XP028754603**
- **COBELLI ET AL.: 'Diabetes: Models, signals, and Control' IEEE REVIEWS IN BIOMEDICAL ENGINEERING vol. 2, 01 January 2009, pages 54 - 96, XP011285638**
- **STÅHL ET AL.: 'Post-prandial plasma glucose prediction in type I diabetes based on Impulse Response Models' IEEE ENGINEERING IN MEDICINE AND BIOLOGY SOCIETY 31 August 2010, pages 1324 - 1327, XP032108407**
- **YAMAMOTO NOGUCHI ET AL.: 'Mathematical model of glucose- insulin metabolism from carbohydrates in type 1 diabetes during postprandial and postabsorptive states' THE SICE ANNUAL CONFERENCE 14 September 2013, pages 170 - 175, XP032566378**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 3 359 039 B1

- **STÅHL ET AL.: 'Predicting Nocturnal Hypoglycemia Using a Non- parametric Insulin Action Model' IEEE INTERNATIONAL CONFERENCE ON SYSTEMS, MAN, AND CYBERNETICS pages 1583 - 1588, XP032847366**

**Description**

Field of the invention

The present invention relates in general to insulin therapy and to the determining of patient-specific physiological and pharmacokinetic parameters related to the glucose-lowering effect of insulin from data collected from the individual, and how to utilize these parameters to improve the insulin therapy outcome.

Technical Background

Globally, 300 to 400 million people have developed diabetes. In 2035 the number is expected to be closer to 600 million. About 5-10% of the diabetic population have type I diabetes and require intensive insulin treatment, and a majority with type II diabetes also require insulin 6-10 years post diagnosis. Modern insulin therapy consists of multiple daily injections using an insulin pen (MDI) or by means of continuous infusion by an insulin pump (CSII). To assess the glucose level personal glucose meters (SMBG), providing a reading of the plasma glucose level at the time of measurement, as well as the evolving continuous glucose measurement systems (CGM), which give frequent measurements (typically every 5 minutes) of the glucose level in the subcutaneous tissue where the sensor is placed, are used. Poor glycemic control due to excessive or insufficient insulin doses may result in both short-term and long-term complications, such as, e.g., acute seizures and coma due to hypoglycemia (low blood glucose concentration) or, e.g., chronic renal disease, blindness and micro- and macrovascular diseases due to long-term hyperglycemia (high blood glucose concentration).

Generally, the diabetes population has unsatisfying glycemic levels and there is a large demand for means to improved control. To optimize the insulin therapy for each individual, knowledge of the glucose-lowering effect and the specific dose requirements are essential. However, current practice and technology do not provide means to effectively and reliably determining these factors on an individual basis.

US 2005/272640 relates to computer implemented method and associated apparatus for the combined control of insulin bolus dosing and basal delivery for the goal of achieving normal glycemic response to meals, exercise, stressors, and other perturbations to blood glucose levels.

Summary of the invention

The invention is as specified in the claims.

The present disclosure provides a method for determining a personalized estimate, the magnitude of an intended medical effect, a duration and a shape of this, for a drug, based on sections of data where a target biomarker and dose history have been recorded, wherein the personalized estimate of a finite impulse response model $a = [a_0, a_1 \dots a_n]$ and the basal hepatic glucose production $G_b$ is determined according to:

$$y(t_k)^{(j)} = y(t_{k-1})^{(j)} + \sum_{i=0}^{n} a_i(y(t_k)^{(j)})I(t_{k-i}) + \qquad (1)$$

$$G_b^{(j)} + v(t_k), \quad t_k \in T_j$$

$I(t_k)$ represents a drug infusion and $y(t_k)^{(j)}$ is a biomarker level at a time sample $tk$, $v(t_k) \sim N(0, \sigma_v)$ corresponds to a process noise perturbation with variance $\sigma_v^2$, and $T_j$ represents the time instances in a dataset $j$, and where impulse-response model parameters $a = [a_0, a_1 \dots a_n]$ have a biomarker dependence;

for each biomarker level G of interest, the following problem is solved to retrieve the parameter estimates; $a$ and $G_b = [G_b^{(1)} \dots G_b^{(N)}]$ using $N$ periods of data:

$$\{a(G), G_b\} = \arg\min \sum_{j=1}^{N} (\|y^{(j)} - \hat{y}^{(j)}\|_{W^{(j)}} + \alpha\|a\|_R$$

$$+ \beta\|G_b^{(j)} - G_b^0\|_2) + \gamma\|a\|_1 \qquad (2)$$

subject to

$$a_k \leq 0, \quad k = [1, \ldots, n-1]$$
$$a_0 = 0$$
$$a_n = 0$$

$$(3)$$

and wherein a second-order regularization matrix R is populated as follows:

$$R(j, j-1 : j+1) = [1 \ -2 \ 1], \quad j = [1, \ldots, n]$$

$$(4)$$

wherein

$$\mathbf{y}^{(j)} = [y_{t_1^j} \cdots y_{t_n^j}]$$

is the collected biomarker data for data record j covering sample times

$$\mathbf{T_j} = [t_1^j \cdots t_n^j],$$

and

$$\hat{\mathbf{y}}^{(j)} = [\hat{y}_{t_1^j} \cdots \hat{y}_{t_n^j}]$$

is the corresponding estimate of biomarker level according to the equation (1); $W^G$ is the quadratic kernel matrix defining the weight of each biomarker measurement according to the distance to the biomarker level $G$; $G_b^0$ is the prior expected value estimate of $G_b$, and $\alpha$, $\beta$ and $\gamma$ are penalty terms.

In one embodiment the method further comprises a step of estimating a required dosage needed, during different time intervals, to achieve an intended effect of the drug on the biomarker.

In one embodiment the method comprises a step of calculating the sensitivity factor of the drug for different glucose values G by summing up all the $a(G) = [ao,(G) \ a_1 (G)... \ a_n(G)]$ terms given by the method according to the present disclosure.

In one embodiment the method comprises calculating an average sensitivity factor of the drug by averaging the sensitivity factors of the above.

In one embodiment the method comprises a step of calculating a drug dose based on the sensitivity factor, the shape and duration of the drug action, information about previous doses Dj at times Tj, the current biomarker level G and target biomarker level Gt according to:

$$D = \max(0, \frac{G - G_t - K_{ISR} \cdot D_{IOB}}{K_{ISR}})$$

$$(8)$$

and

$$D_{IOB} = \sum_{j=1}^{m} D_j \left(1 - \frac{\sum_{i=1}^{(T-T_j)/5} \bar{a}_i}{K_{ISR}}\right), \quad \forall j : T_j \le T - 5 \cdot n \tag{9}$$

In one embodiment the method comprises the steps of using the parameters estimated together with information of the planned dosage and measurements of the biomarker, for calculating an expected effect for a time period covering p sample steps ahead according to:

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \le j \le p \tag{12}$$

where $\hat{y}_{t_k|t_k}$ is the measurement update, $\hat{y}_{t_{k+1}|t_k}$ is the time update, and $\hat{y}_{t_{k+j+1}|t_{k+j}}$ is the predicted value one step ahead, and $\alpha$ is the Kalman filter constant, and $I(t_k)$ is the insulin dose at time $t_k$; and to issue at least one alarm in an electronic device when predefined thresholds are broken.

In one embodiment the method comprises the steps of using the parameters estimated and measurements of the biomarker for simulating the effect of different dosage alternatives for a time period covering p sample steps ahead according to:

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \le j \le p \tag{12}$$

where $\hat{y}_{t_k|t_k}$ is the measurement update, $\hat{y}_{t_{k+1}|t_k}$ is the time update, and $\hat{y}_{t_{k+j+1}|t_{k+j}}$ is the predicted value one step ahead, and $I(t_k)$ is the insulin dose at time $t_k$ and $\alpha$ is the Kalman filter constant.

In one embodiment the method comprises the steps of using the parameters estimated and measurements of the biomarker for simulating the effect of different dosage alternatives for a time period covering p sample steps ahead according to:

$$\hat{y}_{t_k|t_k} = (1-\alpha)\hat{y}_{t_k|t_{k-1}} + \alpha y_t \qquad (10)$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \qquad (11)$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \leq j \leq p \qquad (12)$$

where $\hat{y}_{t_k|t_k}$ is the measurement update, $\hat{y}_{t_{k+1}|t_k}$ is the time update, and $\hat{y}_{t_{k+j+1}|t_{k+j}}$ is the predicted value one step ahead, $I(t_k)$ is the insulin dose at time $t_k$, and $\alpha$ is the Kalman filter constant; and for optimizing a therapy, where $I_S=[I_T \dots I_{T+L}]$ is the suggested insulin dose at the time point of calculation (sample number $T$) and $L(\leq p)$ samples ahead according to:

$$\min_{I_S} C(\hat{Y} - Y_R) \qquad (13)$$

$$\hat{y}_j \geq \gamma_{low}, \quad j = [T \dots T+L] \qquad (14)$$

where C is a convex cost function, $\hat{Y} = [\hat{y}_T \dots \hat{y}_{T+L}]^T$ is the simulated biomarker trace calculated according to the equations (10)-(12), $Y_R = [y_R \dots y_R]$ is a vector the same size as $\hat{Y}$ of the target biomarker value $y_R$.

In one embodiment the method comprises the steps of using the parameters estimated with information about the planned dosage and measurements of the biomarker, for calculating an expected effect and for reprogramming planned doses in a drug delivery pump when predefined thresholds are broken, wherein the new reprogramming dose $I_S=[I_T \dots I_{T+L}]$ at the time point of calculation (sample number $T$) and $L(\leq p)$ samples ahead is based on an optimization according to:

$$\min_{I_S} C(\hat{Y} - Y_R) \qquad (13)$$

subject to

$$\hat{y}_j \geq \gamma_{low}, \quad j = [T \dots T+L] \qquad (14)$$

where C is a convex cost function, $\hat{Y} = [\hat{y}_T \dots \hat{y}_{T+L}]^T$ is the simulated biomarker according to Eq (10-12) trace, $Y_R = [y_R \dots y_R]$ is a vector the same size as $\hat{Y}$ of the target biomarker value $y_R$.

The disclosure relates to a method for determining a personalized estimate of finite impulse response model $b^r=[b^r_1, b^r_2 \dots b^r_n]$ of a biomarker elevating effect of a meal intake of recipe r, according to a maximum likelihood approach where the total likelihood is maximized:

$$\max_{b,\lambda} \; p(Y,I,M_r,G_b,\lambda,b) \propto p(Y|G_b,\lambda,b) \cdot p(G_b,\lambda) \cdot p(b) \qquad (15)$$

and where Y = [$y_1^{(1)}$_... $y_n^{(1)}$_... $y_1^{(N)}$_$y_n^{(N)}$] is the concatenated glucose reference for all meal instances, calculated according to

$$y(t_k)^{(j)} = y(t_{k-1})^{(j)} + \lambda_j \sum_{i=0}^{n} a_i(y(t_k)^{(j)}) I(t_{k-i})$$
$$+ \sum_{r=1}^{R} \sum_{i=0}^{m} b_i^r M_r^{(j)}(t_{k-i}) + G_b^{(j)} + v(t_k), \quad t_k \in T_j$$

$$(16)$$

where $I(t_k)$ represents the drug infusion at time $t_k$, $M_r(t_k)$ is the meal intake in grams of carbohydrates in recipe r at time sample $t_k$, $v(t_k) \sim N(0,\sigma_v)$ corresponds to a process noise perturbation, with variance $\sigma_v^2$, and $T_j$ represents the time instances in dataset j;

In one embodiment the method comprises the steps of using the parameters estimated together with information of the planned dosage and measurements of biomarker, for calculating the expected effect for a time period covering p sample steps ahead according to:

$$\hat{y}_{t_k|t_k} = (1 - \alpha)\hat{y}_{t_k|t_{k-1}} + \alpha y_t$$

$$(18)$$

$$\hat{y}_{t_{k+1}|t_k} = \hat{y}_{t_k|t_k} + \sum_{i=0}^{n} a_i(y(t_k)) I(t_{k-i}) + \hat{G}_{b,T}$$

$$(19)$$

$$\hat{y}_{t_{k+j}|t_{k+j}} = \hat{y}_{t_{k+j}|t_{k+j-1}} + \sum_{i=0}^{n} a_i(\hat{y}_{t_{k+j}|t_{k+j-1}}) I(t_{k+j-i})$$
$$+ \sum_{r=1}^{R} \sum_{i=0}^{m} b_i^r M_r^{(j)}(t_{k-i}) + \hat{G}_{b,T}, \quad 1 \leq j \leq p$$

$$(20)$$

and to issue at least one alarm in an electronic device when predefined thresholds are broken.

In one embodiment the method comprises the steps of using the parameters estimated and measurements of biomarker for simulating the effect of different dosage alternatives for a time period covering p sample steps ahead according to:

$$\hat{y}_{t_k|t_k} = (1 - \alpha)\hat{y}_{t_k|t_{k-1}} + \alpha y_t$$

$$(18)$$

$$\hat{y}_{t_{k+1}|t_k} = \hat{y}_{t_k|t_k} + \sum_{i=0}^{n} a_i(y(t_k)) I(t_{k-i}) + \hat{G}_{b,T}$$

$$(19)$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \sum_{r=1}^{R}\sum_{i=0}^{m} b_r^i M_r^{(i)}(t_{k-i}) + \hat{G}_{b,T}, \quad 1 \le j \le p \tag{20}$$

In one embodiment the method comprises the steps of using the parameters estimated and recent measurements of biomarker for simulating the effect of different dosage alternatives for a time period covering p sample steps ahead according to:

$$\hat{y}_{t_k|t_k} = (1-\alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \sum_{r=1}^{R}\sum_{i=0}^{m} b_r^i M_r^{(i)}(t_{k-i}) + \hat{G}_{b,T}, \quad 1 \le j \le p \tag{20}$$

and for optimizing a therapy $I_S=[I_T... I_{T+L}]$ at the time point of calculation (sample number $T$) and $L(\le p)$ samples ahead according to:

$$\min_{I_S} C(\hat{\mathbf{Y}} - \mathbf{Y_R}) \tag{13}$$

subject to

$$\hat{y}_j \ge \gamma_{low}, \quad j = [T ... T+L] \tag{14}$$

where C is a convex cost function, $\hat{Y} = [\hat{y}_T ... \hat{y}_{T+L}]^T$ is the simulated biomarker according to Eq (10-12) trace, $Y_R = [y_R... y_R]$ is a vector the same size as $\hat{Y}$ of the target biomarker value $y_R$.

The disclosure also relates to a system for controlling insulin delivery to a patient according to the method described in the present disclosure where the measured biomarker is glucose.

In one embodiment the system may be comprising an insulin pump, a glucose meter, a receiver unit for a continuous glucose meter, a smartphone, a tablet, a computer or any other device that may have the capability to display information to a user and perform the necessary measurements and calculations.

The disclosure also relates to a medical device performing the calculations as specified above.

With the above description in mind, an aspect of some of the embodiments of this disclosure relates to determining parameters used to improve insulin therapy in insulin treated diabetes from collected data from an individual. Using these parameters, the glucose-lowering effect, from the time point of an insulin injection until it no longer can be detected, can be determined specifically for that individual. Additionally, the insulin required to maintain, or reach, a treatment target, such as achieving a predefined glucose level, can be calculated for a given time period. The estimates above can be used together with a measurement of the current glucose level, in a medical device or system, to predict the future glucose level and to issue alarms to the user when the predicted value at a certain time point ahead breaks thresholds, indicating potentially dangerous future low or high glucose values. In these devices or systems, different treatment scenarios can be simulated allowing the user to scrutinize and choose a suitable dosing regime. Specifically, in systems where the insulin dosing can be manipulated such as insulin pumps, the insulin delivery can be suspended based on these prediction, and a new insulin dose for the near future can be programmed to reduce the risk of developing dangerously low or high glucose values.

## Short description of the drawings

Figure 1. Example of a section of collected glucose and insulin infusion data. The data in this example has been sampled with a five minute interval.
Figure 2. Example of estimated insulin action across the glucose range.
Figure 3. Example of estimated insulin requirements.
Figure 4. Example of an estimated glucose independent insulin action curve.
Figure 5. Summary of the steps in process to retrieve the parameter estimates.

## Detailed description of the invention

Embodiments of the present disclosure will be described more fully hereinafter with reference to the accompanying drawings, in which embodiments of the disclosure are shown. The disclosure may, however, be embodied in many different forms and should not be construed as limited to the embodiments set forth herein. Rather, these embodiments are provided so that this disclosure will be thorough and complete, and will fully convey the scope of the disclosure to those skilled in the art. Like reference signs refer to like elements throughout.

Embodiments of the present disclosure relate, in general, to the field of insulin therapy. By the term "insulin action" is meant the combined pharmacokinetic and pharmacodynamic metabolic glucose-lowering effect of a specific insulin type for a specific individual.

The pharmacokinetic and pharmacodynamics properties of different insulin types are based on generic assumptions derived from population results, and do not necessarily reflect any particular individual. Using these generic estimates may therefore deviate considerably when applied to a given individual, with potentially poor and dangerous treatment outcomes as result. To mitigate this situation, some additional heuristic rules-of-thumb have been derived. In terms of how to determine the total glucose-lowering effect-the insulin sensitivity factor (ISF)-different formulas exist, but the most widely used is the so-called the 100-rule (or 1800-rule if the mg/dl scale is used). This rule suggests that the ISF can be calculated by dividing 100 by the amount of the total daily insulin dose (TDD). Recently these rules were revised by the originators with slight adjustments to the 100-rule (one author suggests using 109 and the other to use 95). Again, this rule is generic and generally produces poor estimates, and many patients complement this rule by estimating the ISF from personal experience, e.g., from occasional correction boluses. In terms of determining the duration of the metabolic effect no existing method is available. For patients who look for guidance to healthcare professional organizations online often encounter quite short suggested duration of the insulin action, despite that numerous clamp studies suggest that the duration may be up to 8 hours. The American Diabetes Association (ADA) online patient information suggests that the rapid acting insulins peak in about an hour and that the total duration is 2-4 hours. National Institutes of Health (NIH) suggests a peak somewhere between 30 and 90 minutes and a total duration of 3 to 5 hours. Group HealthCare, a non-profit member-owned healthcare organization based in Seattle, suggests a peak at 90 minutes and a total duration of 3 hours. Joslin Diabetes Center advices 30 min - 3 hour peak and a total duration of 3-5 hours. Similar information is provided by NovoNordisk; maximum effect in-between 1 and 3 hours and a total duration of 3-5 hours.

Apparently there is quite a spread in the suggested duration and this reflects the variability in the population, as different clamp studies have suggested. However, for the individual the insulin action duration as well as the shape thereof, are crucial parameters to determine. The shape determines the glucose-lowering effect for each time sample of the total duration from the time point of injection until the time point when the effect is no longer detectable. Mismatch between the expected duration and shape and the true effect and corresponding true shape may result in, e.g., stacking of doses and unexpected hypoglycemia.

The shape of the insulin action is equivalent to a functional description of the dynamic glucose-lowering effect over time of a subcutaneously administered injection of a predefined amount of insulin.

Many modern insulin pumps, as well as some glucose meters, offer decision support systems (DSS) for insulin therapy to the user in the form of so-called bolus guides. These calculators can be used to determine insulin doses based on the current glucose level, a treatment target, the insulin sensitivity factor and the amount of remaining insulin from previous injections. In these systems, the user may select the insulin sensitivity factor, e.g. by the rule presented above, as well as the duration of the insulin action.

The shape of the insulin action profile is sometimes linear, i.e., the glucose-lowering effect is considered constant over the active period, and in some cases a nonlinear curve is used to better reflect the glucose-lowering effect over the action period. As stressed above, neither the magnitude of the glucose-lowering effect nor the dynamics thereof are properly determined for each individual with the currently available methods, reducing the efficacy of these decision supports.

The present disclosure relates to determining patient-specific physiological and pharmacokinetic parameters related to the glucose-lowering effect of insulin from data collected from said individual, and how to utilize these parameters to improve the insulin therapy outcome. Specifically, it is disclosed a method of how to estimate the insulin sensitive factor for a given person and insulin type, as well as the duration and shape of the glucose-lowering effect. Furthermore, the method also provides an estimate of the insulin requirement for this person at different time instances or occasions. Using these calculated estimates together with recent glucose measurements for example in a computer or any other device with calculating capabilities, the future glucose level may be simulated hours in advance. Thus, the method may be used to warn the patient of impeding dangerous low or high glucose values, and different therapy adjustments may be simulated in advance. The description below is based upon that health related data have been collected from an individual treated with insulin and made available, preferably in a digital format. The health related data may cover one or more of: insulin injections of the different insulin types the individual uses, glucose readings from a personal glucose meter and/or continuous glucose sensor or from any other means to measure the capillary, venous or interstitial glucose level. All glucose readings will hereafter be referred to as simply 'glucose'.

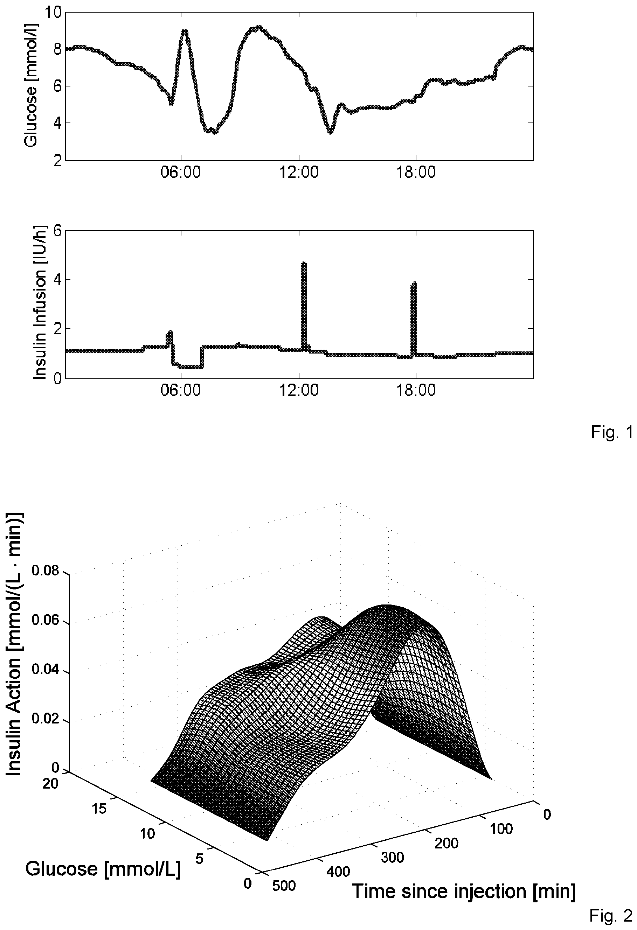

In an embodiment of the present disclosure, the insulin action can be estimated from a record including $N$ different sections of combined insulin dose and glucose data from an individual. An example of collected data can be seen in Fig. 1. These data sections may represent different time frames, e.g. each section represents a specific time section of the day and the entire record covers such sections from couple of weeks or months of data.

A black-box Finite Impulse Response (FIR) model is considered to describe the insulin action, allowing for heterogeneous effects of the insulin action across the glucose range, i.e., higher or lower glucose-lowering effect depending on the current glucose level. However, the fasting glucose dynamics depends on internal dynamics, related to the hepatic glucose production and fasting metabolism, as well as the externally provided insulin. In this approach, this was summarized into a total net basal endogenous glucose production $G_b$ in fasting state. In total, the glucose dynamics during fasting at time point $t_k$ after may then be described as

$$y(t_k)^{(j)} = y(t_{k-1})^{(j)} + \sum_{i=0}^{n} a_i(y(t_k)^{(j)})I(t_{k-i}) + \\ G_b^{(j)} + v(t_k), \quad t_k \in T_j \tag{1}$$

where $I(t_k)$ represents the insulin infusion and $y(t_k)^{(i)}$ is the glucose level at time sample $tk$ in section $j$, $v(tk) \sim N(0, \sigma_v)$ corresponds to a process noise perturbation, with variance $\sigma_v^2$, and $T_j$ represents the time instances in data set $j$, and where the impulse-response model parameters $a = [a_1, a_2... a_n]$ have a glucose dependence. The sampling interval is generally five minutes, but other sampling schemes may also be considered. To estimate the model, e.g., locally-weighted least squares using a quadratic Epaneichnikov kernel or some other kernel may be employed.

In order to keep the estimate smooth, e.g., second-order regularization may also be considered, utilizing e.g. a Gaussian prior for $G_b$. To reduce the model size, the parameters may be regularized by the 1-norm. To fulfill the physiological requirements of glucose-lowering response to insulin, the parameters are constrained to non-positive numbers, and the start and end of the insulin action are enforced to zero. For each glucose level G of interest, the following optimization problem is solved to retrieve the parameter estimates; $a = [a_0, a_1 ... a_n]$ and $G_b = [G_b^{(1)}... G_b^{(N)}]$ using $N$ periods of data.

$$\{\mathbf{a}(G), \mathbf{G_b}\} = \arg\min \sum_{j=1}^{N} (\|\mathbf{y}^{(j)} - \hat{\mathbf{y}}^{(j)}\|_{W^G} + \alpha\|\mathbf{a}\|_R$$
$$+ \beta\|G_b^{(j)} - G_b^0\|_2) + \gamma\|\mathbf{a}\|_1 \tag{2}$$

subject to

$$a_k \leq 0,, \quad k = [1,\ldots,n-1]$$
$$a_0 = 0$$
$$a_n = 0 \tag{3}$$

and where the second-order regularization matrix R is populated as follows:

$$R(j, j-1 : j+1) = [\,1 \;\; -2 \;\; 1\,], \quad j = [1,\ldots,n] \tag{4}$$

Here,

$$\mathbf{y}^{(j)} = [y_{t_1} \cdots y_{t_n}]$$

is the collected glucose data for data record j covering sample times

$$\mathbf{T_j} = [t_1^j \cdots t_n^j],$$

and

$$\hat{\mathbf{y}}^{(j)} = [\hat{y}_{t_1} \cdots \hat{y}_{t_n}]$$

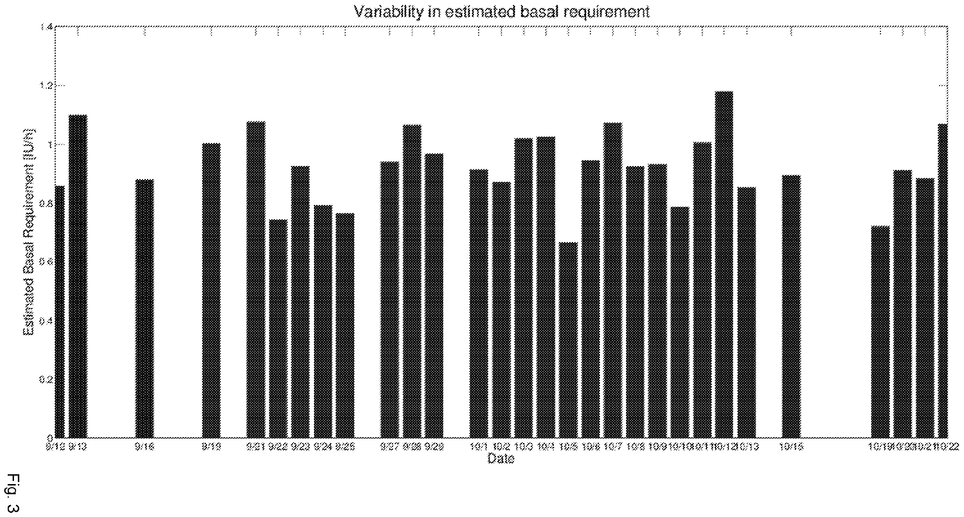

is the corresponding estimate according to Eq. (1). $W^G$ is the quadratic kernel matrix defining the weight of each glucose measurement according to the distance to the glucose level $G$. $G_b^0$ is the prior expected value estimate of $G_b$, and $\alpha$, $\beta$ and $\gamma$ are penalty terms. An example of the insulin action can be seen in Fig. 2 and the $G_b$ is exemplified in Fig. 3.

The total stationary glucose-lowering effect, the insulin sensitivity factor $K_{ISR}$ at a given glucose level $y_b$ is then:

$$\sum_{i=1}^{n} a_i(y_b) \cdot I_b = K_{ISR}(y_b) \tag{5}$$

It is also possible to calculate the mean insulin sensitivity factor over the $n_G$ different glucose values.

$$\bar{K}_{ISR} = \sum_{i=1}^{n_G} \frac{K_{ISR}(y_i)}{n_G}$$

(6)

The mean value may be used as a useful approximation of the glucose-lowering effect independent of the glucose value. Likewise, the finite impulse response parameters may also be averaged over the glucose range to get a glucose independent insulin action curve.

$$\bar{a}_j = \sum_{i=1}^{n_G} \frac{a_j(y_i)}{n_G}, \quad j = [1 \dots n]$$

(7)

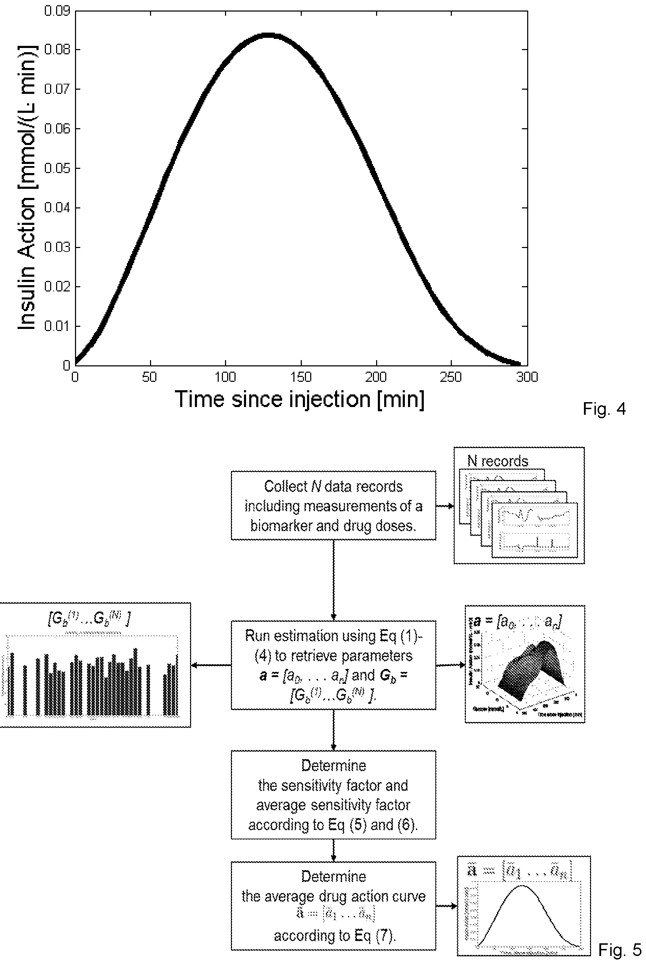

For an example se Fig. 4.

The estimation may be undertaken anew when new data has been collected, thereby assuring that the estimates are up to date. The frequency of re-estimation may vary depending on data availability, clinical practice, and personal conditions. The steps have been summarized in Fig. 5.

The estimates may be used for decision support for insulin bolus dose calculation for both insulin pump and insulin pen therapy. In a bolus calculator, the suggested bolus dose $D$ at time $T$ (rounded to closest 5 minute interval) needed to reach the glucose target $G_t$, considering $m$ previous doses $D_j$ $(j=1...n)$ taken at time point $T_j$ within the time frame of the insulin action is calculated as:

$$D = \max(0, \frac{G - G_t - \bar{K}_{ISR} \cdot D_{IOB}}{\bar{K}_{ISR}})$$

(8)

where $D_{IOB}$ is the amount of active insulin remaining from the previous doses:

$$D_{IOB} = \sum_{j=1}^{m} D_j (1 - \frac{\sum_{i=1}^{(T-T_j)/5} \bar{a}_i}{K_{ISR}}), \quad \forall j : T_j \leq T - 5 \cdot n$$

(9)

The calculated values may be available in an insulin pump, a glucose meter, in a receiver unit for a continuous glucose meter, in smartphones, tablets, computers or other devices that may be used to display information to a user.

In an embodiment of the present disclosure the method described may be used to get estimates of $G_b$, which describe the underlying basal insulin requirement. The estimates may reveal diurnal patterns, day-to-day variations and variations over longer trends. This information may prove useful in clinical practice to enable long-term trend analysis of the patient's health condition, understanding of drivers of glucose variability and barriers to achieving treatment targets, and ultimately a tool for evaluation of changes to the current therapy plan. Paired with monitoring of physical activity, correlation analysis may be undertaken to find causality connections between the level of physical activity and the effect on insulin requirement, thereby enabling preventive action to be undertaken, e.g. to temporarily reduce the insulin doses or reprogram the basal dose regime to avoid hypoglycemic events. These estimates may be made available in analysis software in a computer, smartphone, tablet or other devices that may be used to display information to a user.

In an embodiment of the present disclosure the method may also be used to assess the risk of developing hypo- or hyperglycemia in the near future. Considering the recent insulin dosage and glucose history, and a prior $G_b^0$, a posterior estimate of a scalar $G_b$ for this time period is calculated by maximizing the likelihood $p(G_b/\textbf{Y},\textbf{I},G_b^0)$, where $\textbf{Y} = [y_{T-k}...y_T]$ is the recent glucose history at time $T$ looking $k$ sampling steps back, and $\textbf{I}= [I_{T-k}... I_T]$ is the corresponding recent insulin usage history. Depending on the distribution of the prior, a closed form expression may exist for the estimate. Otherwise, e.g. sequential Monte Carlo methods may be applied. Then, assuming the patient will follow the planned basal regime and based on new glucose measurements $y_t$, the expected glucose trace for the coming hours may be calculated by applying, e.g., a Kalman filter:

$$\dot{\hat{y}}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \hat{G}_{b,T}, \quad 1 \leq j \leq p \tag{12}$$

Where $y_{t_k|t_k}$ is the measurement update, is the time update, and $\hat{y}_{t_{k+j+1}|t_{k+j}}$ is the predicted value one step ahead, and $\alpha$ is the Kalman filter constant.

The normal glycemic range is defined by an upper and lower limit. If the simulated value falls outside these predefined thresholds, alarms are issued to notify the user of the impeding hypo- or hyperglycemia to allow for preventive actions to be taken. To guide the user, suggestions of suitable actions to mitigate the situation may also be provided. These suggestions may be based on reducing or increasing the basal insulin delivery (for insulin pump users) or reduced basal dose (for insulin pen users), and in the case of hyperglycemia of additional correction bolus doses $I_S=[I_T...I_{T+L}]$, where $I_S$ is the suggested insulin dose at the time point of calculation (sample number $T$) and $L(\leq p)$ samples ahead, according to the following optimization routine:

$$\min_{I_S} C(\hat{Y} - Y_R) \tag{13}$$

Subject to

$$\hat{y}_j \geq \gamma_{low}, \quad j = [T...T+L] \tag{14}$$

where C is a convex cost function, $\hat{Y} = [\hat{y}_T ... \hat{y}_{T+L}]^T$ is the simulated glucose trace, $Y_R=[y_R ... y_R]$ is a vector the same size as $\hat{Y}$ of the target glucose value $y_R$, and where the constraint strictly enforces that no hypoglycemia may occur (hypoglycemic threshold $y_{low}$). An example is found in Fig. 6. If no solution is found due to constraint violation (infeasible problem), the user is informed of this and no insulin dose suggestion is provided. Such a warning algorithm may be utilized in an insulin pump, a glucose meter, in a receiver unit for a continuous glucose meter, in smartphones, tablets, computers or other devices that may be used to display information to a user.

In yet another embodiment of the present disclosure the warning algorithm may be extended to not only deliver alarms, but also to allow for the user to simulate the effect of changes to the insulin therapy on the expected glucose trace for the coming hours by exchanging the insulin doses /with the new planned $I_{new}$ in Eq (11) and (12). Thereby the user may assess the risk of different treatment scenarios at his/her own discretion.

In yet another embodiment of the present disclosure the estimated values may be used in an algorithm to reduce or shutoff insulin delivery in an insulin pump. When the predicted glucose value falls below the predefined hypoglycemic threshold with the current therapy plan as calculated by Eq. (10-12), the insulin delivery may be reduced or completely shut off as determined by performing an optimization according to Eq (13-14).

The method described above is not in any way limited to the use of insulin. Dose calculations for other drugs, self-medicated or not, may also benefit from this method to understand the dose response better and thereby enable better tailored doses to each individual. In these cases, the variable correspondences in the equations above are exchanged accordingly, as obvious to someone skilled in the art.

The present disclosure, as described by the different embodiments in conjunction with equations 1 to 14 above, may also be implemented in a system or device for controlling insulin delivery to a patient. Such a system or device may for instance be an insulin pump, a glucose meter, a receiver unit for a continuous glucose meter, a smartphone, a tablet, a computer or any other device that may have the capability to display information to a user and perform the necessary measurements and calculations described in conjunction with equations 1 to 14 in the above text. Many modern insulin pumps, as well as some glucose meters, offer decision support systems (DSS) for insulin therapy to the user in the form of so-called bolus guides. These calculators can be used to determine insulin doses based on the current glucose level, a treatment target, the insulin sensitivity factor and the amount of remaining insulin from previous injections. In these systems, the user may select the insulin sensitivity factor, e.g. by the method presented in equations 1 to 14, as well as the duration of the insulin action.

According another aspect of the disclosure, a computer implemented method for managing blood glucose is provided. The method comprises the step of estimating the required insulin dosage needed, during different time intervals, to achieve normoglycemia. The computer implemented method further comprising the step of calculating the sensitivity factor of the drug according to Eq. (6) and the shape and duration of the drug action according to Eq. (7). Finally, comprising the step of calculating a dose according to Eq. (8) and (9) based on the sensitivity factor, the shape and duration of the drug action, information about previous doses, the current biomarker level and target biomarker level.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms used herein should be interpreted as having a meaning that is consistent with their meaning in the context of this specification and the relevant art and will not be interpreted in an idealized or overly formal sense unless expressly so defined herein.

This model according to Eq (1) can be extended with another FIR model, which represents the net glucose-elevating effect, the meal impact, following a meal. By incorporating the insulin gain $\lambda$, the glucose dynamics at time point $t_k$, during meal instance j, may then be described as:

$$y(t_k)^{(j)} = y(t_{k-1})^{(j)} + \lambda_j \sum_{i=0}^{n} a_i(y(t_k)^{(j)}) I(t_{k-i})$$
$$+ \sum_{r=1}^{R} \sum_{i=0}^{m} b_i^r M_r^{(j)}(t_{k-i}) + G_b^{(j)} + v(t_k), \quad t_k \in T_j$$

$$(15)$$

where $I(t_k)$ represents the insulin infusion, $M_r(t_k)$ is the meal intake in grams of carbohydrates in recipe r, and $y(t_k)$ is the glucose level at time sample $t_k$, $v(t_k) \sim N(0,\sigma_v)$ corresponds to a process noise perturbation, with variance $\sigma_v^2$, and $T_j$ represents the time instances in dataset j. The meal impact parameters $\mathbf{b}^r = [b_1^r, b_2^r ... b_n^r]$ are fixed for each recipe. In order to fulfill physiologically qualitatively correct responses, constraints were imposed on the FIR parameters. Just as the parameters of the insulin action FIR were restricted to non-positive numbers, the parameters of the meal impact model were restricted to non-negative numbers. A recipe is a unique combination of ingredients, and may denote a single ingredient. Also note that the recipes are specific to an individual (i.e., two persons eating a banana constitutes two different recipes). The net basal endogenous glucose production $G_b$ and the insulin multipliers are allowed to vary between different meal instances to capture variations in insulin sensitivity. The insulin action model was considered fixed with the parameter values given by the estimation of that model. To estimate $\mathbf{b}^r$, $\mathbf{G_b} = [G_b^{(1)} ... G_b^{(N)}]$ and $\lambda = [\lambda^{(1)} ... \lambda^{(N)}]$ for N number of meal instances, a maximum-likelihood approach is considered. The total likelihood for the entire dataset from all the meal instances is:

$$p(Y, I, M_r, G_b, \lambda, b) \propto p(Y|G_b, \lambda, b) \cdot p(G_b, \lambda) \cdot p(b)$$

$$(16)$$

where $\mathbf{Y} = [y_1^{(1)}\_\ldots y_n^{(1)}\_\ldots y_1^{(N)}\_y_n^{(N)}]$ is the concatenated glucose reference for all meal instances. We would like to maximize this in order to retrieve our parameter estimates. Different priors may be utilized as deemed appropriate. One method among others, a Gibbs sampler, where samples are drawn in an iterative scheme from the conditional distributions may be used to retrieve the parameter estimates:

$$(G_b, \lambda)^{(k)} \sim p(G_b, \lambda | \mathbf{Y}, \mathbf{b}^{(k-1)})$$
$$b_j^{(k)} \sim p(b_j | \mathbf{Y}, \mathbf{b}_{\neg j}^{(k)}, (G_b, \lambda)^{(k)}), \quad \forall b_j \in \mathbf{b} \tag{17}$$

where k is the sampling index and

$$\mathbf{b}_{\neg j}^{(k)}$$

is the meal impact vector without term $b_j$. When a sufficient number of samples have been collected the expected values can be retrieved. A suitable number of iterations may be determined by someone skilled in the art. The samples are restricted to positive numbers to fulfill the physiological constraints. Using the augmented model, the glucose may be predicted, using the same notation as in Eq (10-12,15):

$$\hat{y}_{t_k|t_k} = (1 - \alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \sum_{r=1}^{R} \sum_{i=0}^{m} b_r^i M_r^{(i)}(t_{k-i})$$
$$+ \hat{G}_{b,T}, \quad 1 \le j \le p \tag{20}$$

Likewise, Eq (13-14) may be updated with the augmented model according to Eq (15) to recommend doses.

**Claims**

1. A device configured to perform calculations for determining a personalized estimate; the magnitude of an intended medical effect, a duration and a shape of this, for a drug, based on sections of data where a target glucose and dose history have been recorded, wherein the personalized estimate of a finite impulse-response model parameters $\mathbf{a} = [a_0, a_1 \ldots a_n]$ and the basal hepatic glucose production $G_b$ is determined according to:

$$y(t_k)^{(j)} = y(t_{k-1})^{(j)} + \sum_{i=0}^{n} a_i(y(t_k)^{(j)})I(t_{k-i}) +$$

$$G_b^{(j)} + v(t_k), \quad t_k \in T_j \tag{1}$$

$I(t_k)$ represents a drug infusion and $y(t_k)^{(i)}$ is a glucose level at a time sample $t_k$, $v(t_k) \sim N(0, \sigma_v)$ corresponds to a process noise perturbation with variance $\sigma_v^2$, and $T_j$ represents the time instances in a dataset j, and where the finite impulse-response model parameters $a = [a_0, a_1 \ldots a_n]$ have a glucose dependence;

for each glucose level $G$ of interest, the following problem is solved to retrieve the parameter estimates; $a$ and $G_b = [G_b^{(1)} \ldots G_b^{(N)}]$ using $N$ periods of data:

$$\{\mathbf{a}(G), \mathbf{G_b}\} = \arg\min \sum_{j=1}^{N} (\|\mathbf{y}^{(j)} - \hat{\mathbf{y}}^{(j)}\|_{W^G} + \alpha\|\mathbf{a}\|_R$$

$$+ \beta\|G_b^{(j)} - G_b^0\|_2) + \gamma\|\mathbf{a}\|_1 \tag{2}$$

subject to

$$a_k \leq 0, , \quad k = [1, \ldots, n-1]$$
$$a_0 = 0$$
$$a_n = 0 \tag{3}$$

and wherein a second-order regularization matrix $R$ is populated as follows:

$$R(j, j-1 : j+1) = \begin{bmatrix} 1 & -2 & 1 \end{bmatrix}, \quad j = [1, \ldots, n] \tag{4}$$

wherein $\mathbf{y^{(j)}} = [y_{t_1^j} \cdots y_{t_n^j}]$ is the collected glucose data for data record j covering sample times $\mathbf{T_j} = [t_1^j \ldots t_n^j]$, and $\hat{\mathbf{y}}^{(j)} = [\hat{y}_{t_1^j} \cdots \hat{y}_{t_n^j}]$ is the corresponding estimate of glucose level according to the equation (1); $W^G$ is the quadratic kernel matrix defining the weight of each glucose measurement according to the distance to the glucose level $G$; $G_b^0$ is the prior expected value estimate of $G_b$, and $\alpha$, $\beta$ and $\gamma$ are penalty terms.

2. The device according to claim 1, further configured to estimate a required dosage needed, during different time intervals, to achieve an intended effect of the drug on the glucose.

3. The device according to any one of claims 1 or 2, further configured to calculate the sensitivity factor of the drug for different glucose values G by summing up all the $a(G) = [a_0,(G) a_1(G)\ldots a_n(G)]$ terms given by the method according to claim 1.

4. The device according to claim 3, further configured to calculate an average sensitivity factor of the drug by averaging the sensitivity factors of claim 3.

5. The device according to claim 4, further configured to calculate a drug dose based on the sensitivity factor, the shape and duration of the drug action, information about previous doses Dj at times Tj, the current glucose level G and target glucose level Gt according to:

$$D = \max\left(0, \frac{G - G_t - \bar{K}_{ISR} \cdot D_{IOB}}{\bar{K}_{ISR}}\right) \tag{8}$$

and

$$D_{IOB} = \sum_{j=1}^{m} D_j \left(1 - \frac{\sum_{i=1}^{(T-T_j)/5} \bar{a}_i}{\bar{K}_{ISR}}\right), \quad \forall j : T_j \leq T - 5 \cdot n \tag{9}$$

6. The device according to any one of the previous claims, configured to use the parameters estimated according to claim 1 together with information of the planned dosage and measurements of the glucose, for calculating an expected effect for a time period covering p sample steps ahead according to:

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \leq j \leq p \tag{12}$$

where $\hat{y}_{t_k|t_k}$ is the measurement update, $\hat{y}_{t_k|t_k}$ is the time update, and $\hat{y}_{t_{k+j+1}|t_{k+j}}$ is the predicted value one step ahead, and $\alpha$ is the Kalman filter constant, and $I(t_k)$ is the insulin dose at time $t_k$; and to issue at least one alarm in an electronic device when predefined thresholds are broken.

7. The device according to any of the previous claims, configured to use the parameters estimated according to claim 1 and measurements of the glucose for simulating the effect of different dosage alternatives for a time period covering p sample steps ahead according to:

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i\left(\hat{y}_{t_{k+j}|t_{k+j-1}}\right) I(t_{k+j-i})$$

$$+ \hat{G}_{b,T}, \quad 1 \le j \le p \tag{12}$$

where $\hat{y}_{t_k|t_k}$ is the measurement update, $\hat{y}_{t_k|t_k}$ is the time update, and $\hat{y}_{t_{k+j+1}|t_{k+j}}$ is the predicted value one step ahead, and $I(t_k)$ is the insulin dose at time $t_k$ and $\alpha$ is the Kalman filter constant.

8. The device according to claim 1, configured to use the parameters estimated according to claim 1 and measurements of the glucose for simulating the effect of different dosage alternatives for a time period covering p sample steps ahead according to:

$$\hat{y}_{t_k|t_k} = (1-\alpha)\hat{y}_{t_k|t_k-1} + \alpha y_t \tag{10}$$

$$\hat{y}_{t_{k+1}|t_k} = \hat{y}_{t_k|t_k} + \sum_{i=0}^{n} a_i(y(t_k)) I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i\left(\hat{y}_{t_{k+j}|t_{k+j-1}}\right) I(t_{k+j-i})$$

$$+ \hat{G}_{b,T}, \quad 1 \le j \le p \tag{12}$$

where $\hat{y}_{t_k|t_k}$ is the measurement update, $\hat{y}_{t_k|t_k}$ is the time update, and $\hat{y}_{t_{k+j+1}|t_{k+j}}$ is the predicted value one step ahead, $I(t_k)$ is the insulin dose at time $t_k$, and $\alpha$ is the Kalman filter constant; and for optimizing a therapy, where $I_S=[I_T \ldots I_{T+L}]$ is the suggested insulin dose at the time point of calculation (sample number 7) and $L$ ($\le p$) samples ahead according to:

$$\min_{\mathbf{I_S}} C(\hat{\mathbf{Y}} - \mathbf{Y_R}) \tag{13}$$

subject to

$$\hat{y}_j \ge \gamma_{low}, \quad j = [T \ldots T+L] \tag{14}$$

where C is a convex cost function, $\hat{Y} = [\hat{y}_T \ldots \hat{y}_{T+L}]^T$ is the simulated glucose trace calculated according to the equations (10)-(12), $\mathbf{Y_R} = [y_R \ldots y_R]$ is a vector the same size as $\hat{Y}$ of the target glucose value $y_R$.

9. The device according to any of the previous claims, configured to use the parameters estimated according to claim 1 with information about the planned dosage and measurements of the glucose, for calculating an expected effect and for reprogramming planned doses in a drug delivery pump when predefined thresholds are broken, wherein the new reprogramming dose $I_S=[I_T \ldots I_{T+L}]$ at the time point of calculation (sample number 7) and $L(\le p)$ samples ahead is based on an optimization according to:

$$\min_{\mathbf{I_S}} C(\hat{\mathbf{Y}} - \mathbf{Y_R}) \tag{13}$$

subject to

$$\hat{y}_j \geq \gamma_{low}, \quad j = [T \dots T + L] \tag{14}$$

where C is a convex cost function, $\hat{Y} = [\hat{y}_T \dots \hat{y}_{T+L}]^T$ is the simulated glucose according to Eq (10-12) trace, $\mathbf{Y}_R = [y_R \dots y_R]$ is a vector the same size as $\hat{Y}$ of the target glucose value $y_R$.

**10.** The device according to any of the previous claims, configured to perform calculations for determining a personalized estimate of finite impulse response model parameters $\mathbf{b}^r = [b^r_1, b^r_2 \dots b^r_n]$ of a glucose elevating effect of a meal intake of recipe r, according to a maximum likelihood approach where the total likelihood is maximized:

$$\max{}_{\mathbf{b},\lambda} \quad p(\mathbf{Y}, \mathbf{I}, \mathbf{M}_r, \mathbf{G_b}, \lambda, \mathbf{b}) \propto p(\mathbf{Y}|\mathbf{G_b}, \lambda, \mathbf{b}) \cdot p(\mathbf{G_b}, \lambda) \cdot p(\mathbf{b}) \tag{15}$$

and where $Y = [y_1^{(1)}\_\dots y_n^{(1)}\_\dots y_1^{(N)}\_y_n^{(N)}]$ is the concatenated glucose reference for all meal instances, calculated according to

$$y(t_k)^{(j)} = y(t_{k-1})^{(j)} + \lambda_j \sum_{i=0}^{n} a_i(y(t_k)^{(j)}) I(t_{k-i})$$

$$+ \sum_{r=1}^{R} \sum_{i=0}^{m} b_i^r M_r^{(j)}(t_{k-i}) + G_b^{(j)} + v(t_k), \quad t_k \in T_j \tag{16}$$

where $I(t_k)$ represents the drug infusion at time $t_k$, $M_r(t_k)$ is the meal intake in grams of carbohydrates in recipe r at time sample $t_k$, $v(t_k) \sim N(0, \sigma_v)$ corresponds to a process noise perturbation, with variance $\sigma_v^2$, Tj represents the time instances in dataset j, and where the finite impulse-response model parameters $a$ is as of claim 1.

**11.** The device according to any of the previous claims configured to use the parameters estimated according to claim 1 and 10 together with information of the planned dosage and measurements of glucose, for calculating the expected effect for a time period covering p sample steps ahead according to:

$$\hat{y}_{t_k|t_k} = (1 - \alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k)) I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}}) I(t_{k+j-i})$$

$$+ \sum_{r=1}^{R} \sum_{i=0}^{m} b_i^r M_r^{(j)}(t_{k-i}) + \hat{G}_{b,T}, \quad 1 \leq j \leq p \tag{20}$$

and to issue at least one alarm in an electronic device when predefined thresholds are broken.

12. The device according to any of the previous claims configured to use the parameters estimated according to claim 1 and 10 and measurements of glucose for simulating the effect of different dosage alternatives for a time period covering p sample steps ahead according to:

$$\hat{y}_{t_k|t_k} = (1-\alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p \tag{20}$$

13. The device according to any of the previous claims configured to use the parameters estimated according to any one of claim 1 and 10 and recent measurements of glucose for simulating the effect of different dosage alternatives for a time period covering p sample steps ahead according to:

$$\hat{y}_{t_k|t_k} = (1-\alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p \tag{20}$$

and for optimizing a therapy $I_S = [I_T...I_{T+L}]$ at the time point of calculation (sample number 7) and $L(\le p)$ samples ahead according to:

$$\min_{I_S} C(\hat{Y} - Y_R) \tag{13}$$

subject to

$$\hat{y}_j \ge \gamma_{low}, \quad j = [T...T+L] \tag{14}$$

. where C is a convex cost function, $\hat{Y} = [\hat{y}_T \ldots \hat{y}_{T+L}]^T$ is the simulated glucose according to Eq (10-12) trace, $Y_R = [y_R \ldots y_R]$ is a vector the same size as $\hat{Y}$ of the target glucose value $y_R$.

14. A system comprising the device according to claims 1-13 and further comprising an insulin pump, a glucose meter, a receiver unit for a continuous glucose meter, a smartphone, a tablet, a computer or any other device that may have the capability to display information to a user and perform the necessary measurements and calculations.

**Patentansprüche**

1. Vorrichtung, die ausgestaltet ist zum Durchführen von Berechnungen zum Bestimmen einer personalisierten Schätzung; des Ausmaßes einer vorgesehenen medizinischen Wirkung, einer Dauer und einer Form davon für ein Arzneimittel basierend auf Segmenten von Daten, in denen eine Zielglucose und eine Dosishistorie aufgezeichnet worden sind, wobei die personalisierte Schätzung von Parametern eines Modells mit endlicher Impulsantwort $a = [a_0, a_1 \ldots a_n]$ und der basalen hepatischen Glucoseproduktion $G_b$ bestimmt wird gemäß:

$$y(t_k)^{(j)} = y(t_{k-1})^{(j)} + \sum_{i=0}^{n} a_i(y(t_k)^{(j)}) I(t_{k-i}) +$$

$$G_b^{(j)} + v(t_k), \quad t_k \in T_j \tag{1}$$

wobei $I(t_k)$ eine Arzneimittelinfusion repräsentiert und $y(t_k)^{(j)}$ ein Glucosespiegel zur Zeit der Probenahme $t_k$ ist, $v(t_k) \sim N(0, \sigma_v)$ einer Prozessrauschenperturbation mit der Varianz $\sigma_v^2$ entspricht, und $T_j$ die Zeitinstanzen in einem Datensatz $j$ repräsentiert, und wobei die Parameter des Modells mit endlicher Impulsantwort $a = [a_0, a_1 \ldots a_n]$ eine Glucoseabhängigkeit aufweisen;
für jeden interessierenden Glucosespiegel $G$ das folgende Problem gelöst wird, um die Parameterschätzungen $a$ und $Gb = [Gb^{(1)} \ldots G_b^{(N)}]$ unter Verwendung von N Datenperioden abzurufen:

$$\{a(G), G_b\} = \arg\min \sum_{j=1}^{N} (\|y^{(j)} - \hat{y}^{(j)}\|_{W^G} + \alpha\|a\|_R$$

$$+ \beta\|G_b^{(j)} - G_b^0\|_2) + \gamma\|a\|_1 \tag{2}$$

Gemäß

$$a_k \leq 0,, \quad k = [1, \ldots, n-1]$$
$$a_0 = 0$$
$$a_n = 0 \tag{3}$$

und wobei eine Regulatisierungsmatrix zweiter Ordnung R wie folgt ausgefüllt wird:

$$R(j, \ j-1: \ j+1) = [1 \ -2 \ 1], \quad j = [1, \ \ldots, \ n] \tag{4}$$

wobei $\mathbf{y}^{(j)} = [y_{t_1^j} \ldots y_{t_n^j}]$ die gesammelten Glucosedaten für Datenaufzeichnung j sind, die die Probenahmezeiten $\mathbf{T_j} = [t_1^j \ldots t_n^j]$ abdecken, und $\hat{\mathbf{y}}^{(j)} = [\hat{y}_{t_1^j} \ldots \hat{y}_{t_n^j}]$ die entsprechende Schätzung des Glucosespiegels gemäß der Gleichung (1) ist; $W^G$ die quadratische Kernmatrix ist, die die Gewichtung jeder Glucosemessung gemäß dem Abstand zu dem Glucosespiegel G definiert; $G_b^0$ die frühere erwartete Werteschätzung von $G_b$ ist, und $\alpha$, $\beta$ und $\gamma$ Strafterme sind.

2. Vorrichtung nach Anspruch 1, die des Weiteren ausgestaltet ist, um eine erforderliche Dosis zu schätzen, die während unterschiedlicher Zeitintervalle gebraucht wird, um eine vorgesehene Wirkung des Arzneimittels auf die Glucose zu erreichen.

3. Vorrichtung nach einem der Ansprüche 1 oder 2, die des Weiteren ausgestaltet ist, um den Empfindlichkeitsfaktor des Arzneimittels für unterschiedliche Glucosewerte G zu berechnen, indem alle der Terme $a(G) = [a_0, (G)\ a_1(G)\ ...a_n (G)]$ aufsummiert werden, die durch das Verfahren gemäß Anspruch 1 gegeben sind.

4. Vorrichtung nach Anspruch 3, die des Weiteren ausgestaltet ist, um einen durchschnittlichen Empfindlichkeitsfaktor des Arzneimittels zu berechnen, indem die Empfindlichkeitsfaktoren von Anspruch 3 gemittelt werden.

5. Vorrichtung nach Anspruch 4, die des Weiteren ausgestaltet ist, um eine Arzneimitteldosis basierend auf dem Empfindlichkeitsfaktor, der Form und Dauer der Arzneimittelwirkung, Informationen zu vorhergehenden Dosen $D_j$ zu den Zeitpunkten $T_j$, dem aktuellen Glucosespiegel G und dem Zielglucosespiegel Gt zu berechnen gemäß:

$$D = \max(0, \frac{G - G_t - \bar{K}_{ISR} \cdot D_{IOB}}{\bar{K}_{ISR}}) \tag{8}$$

und

$$D_{IOB} = \sum_{j=1}^{m} D_j(1 - \frac{\sum_{i=1}^{(T-T_j)/5} \bar{a}_i}{K_{ISR}}), \quad \forall j : T_j \leq T - 5 \cdot n \tag{9}$$

6. Vorrichtung nach einem der vorhergehenden Ansprüche, die ausgestaltet ist, um die gemäß Anspruch 1 geschätzten Parameter zusammen mit Informationen zur geplanten Dosierung und Messungen der Glucose zu verwenden, um eine erwartete Wirkung für eine Zeitperiode, die p Probenahmeschritte voraus abdeckt, zu berechnen gemäß:

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \leq j \leq p \tag{12}$$

wobei die Messungsaktualisierung ist, $\hat{y}_{t_{k+1}|t_k}$ die Zeitaktualisierung ist, und $\hat{y}_{t_{k+j+1}|t_{k+j}}$ der vorhergesagte Wert für einen Schritt voraus ist, und $\alpha$ die Kalman-Filterkonstante ist, und $I(t_k)$ die Insulindosis zu der Zeit $t_k$ ist; und um mindestens einen Alarm in einer elektronischen Vorrichtung auszugeben, wenn vordefinierte Schwellenwerte verletzt werden.

7. Vorrichtung nach einem der vorhergehenden Ansprüche, die ausgestaltet ist, um die gemäß Anspruch 1 geschätzten Parameter und Messungen der Glucose zu verwenden, um die Wirkung von verschiedenen Dosierungsalternativen für eine Zeitperiode, die p Probenahmeschritte voraus abdeckt, zu simulieren gemäß:

$$\hat{y}_{t_k|t_k} = (1-\alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i}) \\ + \hat{G}_{b,T}, \quad 1 \le j \le p \tag{12}$$

wobei $\hat{y}_{t_k}|t_k$ die Messungsaktualisierung ist, $\hat{y}_{t_k}|t_k$ die Zeitaktualisierung ist, und $\hat{y}_{t_{k+j+1}}|t_{k+j}$ der vorhergesagte Wert einen Schritt voraus ist, und $I(t_k)$ die Insulindosis zu der Zeit $t_k$ ist, und $\alpha$ die Kalman-Filterkonstante ist.

8. Vorrichtung nach Anspruch 1, die ausgestaltet ist, um die gemäß Anspruch 1 geschätzten Parameter und Messungen der Glucose zu verwenden, um die Wirkung von verschiedenen Dosierungsalternativen für eine Zeitperiode, die p Probenahmeschritte voraus abdeckt, zu simulieren gemäß:

$$\hat{y}_{t_k|t_k} = (1-\alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i}) \\ + \hat{G}_{b,T}, \quad 1 \le j \le p \tag{12}$$

wobei $\hat{y}_{t_k}|t_k$ die Messungsaktualisierung ist, $\hat{y}_{t_{k+1}}|t_k$ die Zeitaktualisierung ist, und $\hat{y}_{t_{k+j+1}}|t_{k+j}$ der vorhergesagte Wert einen Schritt voraus ist, $I(t_k)$ die Insulindosis zu der Zeit $t_k$ ist, und $\alpha$ die Kalman-Filterkonstante ist; und um eine Therapie zu optimieren, wobei $I_s = [I_T...I_{T+L}]$ die vorgeschlagene Insulindosis zum Zeitpunkt der Berechnung (Probenahmenummer T) und $L(\le p)$ Probenahmen voraus ist entsprechend:

$$\min_{I_s} C(\hat{Y} - Y_R) \tag{13}$$

gemäß

$$\hat{y}_j \ge \gamma_{niedrig} \qquad j = [T...T+L] \tag{14}$$

wobei $C$ eine konvexe Kostenfunktion ist, $\hat{Y} = [\hat{y}_T...\hat{y}_{T+L}]^T$ die simulierte Glucosekurve ist, die gemäß den Gleichungen (10)-(12) berechnet wird, $Y_R = [y_R \ldots Y_R]$ ein Vektor mit der gleichen Größe wie $\hat{Y}$ des Zielglucosewerts $y_R$ ist.

9. Vorrichtung nach einem der vorhergehenden Ansprüche, die ausgestaltet ist, um die gemäß Anspruch 1 geschätzten Parameter mit Informationen zu der geplanten Dosierung und Messungen der Glucose zu verwenden, um eine erwartete Wirkung zu berechnen und geplante Dosen in einer Arzneimittelabgabepumpe umzuprogrammieren, wenn vordefinierte Schwellenwerte verletzt werden, wobei die neue Umprogrammierungsdosis $I_s = [I_T \ldots I_{T+L}]$ zum Zeitpunkt der Berechnung (Probenahmenummer T) und $L(\leq p)$ Probenahmen voraus auf einer Optimierung wie folgt basiert:

$$\min_{I_s} C(\hat{Y} - Y_R) \tag{13}$$

gemäß

$$\hat{y}_j \geq \gamma_{niedrig} \qquad j = [T \ldots T+L] \tag{14}$$

wobei C eine konvexe Kostenfunktion ist, $\hat{Y} = [\hat{y}_T \ldots \hat{y}_{T+L}]^T$ die simulierte Glucose gemäß den Kurven der Gleichungen (10-12) ist, $Y_R = [y_R \ldots y_R]$ ein Vektor mit der gleichen Größe wie $\hat{Y}$ des Zielglucosewerts $y_R$ ist.

10. Vorrichtung nach einem der vorhergehenden Ansprüche, die ausgestaltet ist, um Berechnungen zum Bestimmen einer personalisierten Schätzung von Parametern eines Modells mit endlicher Impulsantwort $b^r = [b^r_1, b^r_2 \ldots b^r_n]$ von einer Glucose erhöhenden Wirkung einer Einnahme einer Mahlzeit mit dem Rezept r gemäß einem Ansatz der maximalen Wahrscheinlichkeit durchzuführen, wobei die Gesamtwahrscheinlichkeit wie folgt maximiert wird:

$$\max_{b,\lambda} \; p(Y, I, M_r, G_b, \lambda, b) \propto p(Y|G_b, \lambda, b) \cdot p(G_b, \lambda) \cdot p(b) \tag{15}$$

und wobei $Y = [y_1^{(1)} \_ \ldots y_n^{(1)} \_ \ldots y_1^{(N)} \_ y_n^{(N)}]$ die verkettete Glucosereferenz für alle Mahlzeitinstanzen ist, die berechnet wird gemäß

$$y(t_k)^{(j)} = y(t_{k-1})^{(j)} + \lambda_j \sum_{i=0}^{n} a_i(y(t_k)^{(j)})I(t_{k-i})$$

$$+ \sum_{r=1}^{R} \sum_{i=0}^{m} b_i^r M_r^{(j)}(t_{k-i}) + G_b^{(j)} + v(t_k), \quad t_k \in T_j \tag{16}$$

wobei $I(t_k)$ die Medikamenteninfusion zur Zeit $t_k$ repräsentiert, $M_r(t_k)$ die Einnahme der Mahlzeit in Gramm Kohlenhydrate im Rezept r zur Zeit der Probenahme $t_k$ ist, $v(t_k) \sim N(0, \sigma_v)$ einer Prozessrauschenperturbation mit der Varianz $\sigma_v^2$ entspricht, $T_j$ die Zeitinstanzen im Datensatz j repräsentiert, und wobei die Parameter des Modells mit endlicher Impulsantwort $a$ wie in Anspruch 1 sind.

11. Vorrichtung nach einem der vorhergehenden Ansprüche, die ausgestaltet ist, um die gemäß Anspruch 1 und 10 geschätzten Parameter zusammen mit Informationen zur geplanten Dosierung und Messungen der Glucose zu verwenden, um die erwartete Wirkung für eine Zeitperiode, die p Probenahmeschritte voraus abdeckt, zu berechnen gemäß:

$$\hat{y}_{t_k|t_k} = (1 - \alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i\big(y(t_k)\big)I(t_{k-i}) + \hat{G}_{b,T}$$

(19)

$$\hat{y}_{t_{k+j+1}|t_{k+j}} = \hat{y}_{t_{k+j}|t_{k+j-1}} + \sum_{i=0}^{n} a_i\big(\hat{y}_{t_{k+j}|t_{k+j-1}}\big)I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p$$

(20)

und um mindestens einen Alarm in einer elektronischen Vorrichtung auszugeben, wenn vordefinierte Schwellenwerte verletzt werden.

12. Vorrichtung nach einem der vorhergehenden Ansprüche, die ausgestaltet ist, um die gemäß Anspruch 1 und 10 geschätzten Parameter und Messungen der Glucose zu verwenden, um die Wirkung von verschiedenen Dosierungsalternativen für eine Zeitperiode, die p Probenahmeschritte voraus abdeckt, zu simulieren gemäß:

$$\hat{y}_{t_k|t_k} = (1-\alpha)\hat{y}_{t_k|t_{k-1}} + \alpha y_t$$

(18)

$$\hat{y}_{t_{k+1}|t_k} = \hat{y}_{t_k|t_k} + \sum_{i=0}^{n} a_i\big(y(t_k)\big)I(t_{k-i}) + \hat{G}_{b,T}$$

(19)

$$\hat{y}_{t_{k+j+1}|t_{k+j}} = \hat{y}_{t_{k+j}|t_{k+j-1}} + \sum_{i=0}^{n} a_i\big(\hat{y}_{t_{k+j}|t_{k+j-1}}\big)I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p$$

(20)

13. Vorrichtung nach einem der vorhergehenden Ansprüche, die ausgestaltet ist, um die gemäß einem der Ansprüche 1 und 10 geschätzten Parameter und kürzlich erfolgten Messungen der Glucose zu verwenden, um die Wirkung von verschiedenen Dosierungsalternativen für eine Zeitperiode, die p Probenahmeschritte

$$\hat{y}_{t_k|t_k} = (1-\alpha)\hat{y}_{t_k|t_{k-1}} + \alpha y_t$$

(18)

$$\hat{y}_{t_{k+1}|t_k} = \hat{y}_{t_k|t_k} + \sum_{i=0}^{n} a_i\big(y(t_k)\big)I(t_{k-i}) + \hat{G}_{b,T}$$

(19)

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \sum_{r=1}^{R}\sum_{i=0}^{m} b_i^r M_r^{(j)}(t_{k-i}) + \hat{G}_{b,T}, \quad 1 \leq j \leq p \tag{20}$$

voraus abdeckt, zu simulieren gemäß: und um eine Therapie $I_s = [I_T...I_{T+L}]$ zum Zeitpunkt der Berechnung (Probenahmenummer T) und L($\leq$p) Probenahmen voraus zu optimieren entsprechend:

$$\min_{I_s} C(\dot{Y} - Y_R) \tag{13}$$

gemäß

$$\hat{y}_j \geq \gamma \ \textit{niedrig} \qquad j = [T...T+L] \tag{14}$$

wobei C eine konvexe Kostenfunktion ist, $\hat{Y} = [\hat{y}_T... \hat{y}_{T+L}]^T$ die simulierte Glucose gemäß den Kurven der Gleichungen (10-12) ist, $Y_R = [y_R ... y_R]$ ein Vektor mit der gleichen Größe wie $\hat{Y}$ des Zielglucosewerts $y_R$ ist.

14. System, umfassend die Vorrichtung nach den Ansprüchen 1 bis 13, und des Weiteren umfassend eine Insulinpumpe, ein Glucosemessgerät, eine Empfängereinheit für ein kontinuierliches Glucosemessgerät, ein Smartphone, ein Tablet, ein Computer oder jedwedes andere Gerät, das die Fähigkeit zum Anzeigen von Informationen an einen Anwender und zum Durchführen der notwendigen Messungen und Berechnungen haben kann.

## Revendications

1. Dispositif configuré pour réaliser des calculs pour déterminer une estimation personnalisée ; l'amplitude d'un effet médical escompté, une durée et une forme de celui-ci, pour un médicament, sur la base de sections de données où un glucose cible et un historique de dose ont été enregistrés, l'estimation personnalisée de paramètres d'un modèle de réponse impulsionnelle finie $a = [a_0, a_1 ... a_n]$ et la production de glucose hépatique basal $G_b$ étant déterminée selon :

$$y(t_k)^{(j)} = y(t_{k-1})^{(j)} + \sum_{i=0}^{n} a_i(y(t_k)^{(j)})I(t_{k-i}) +$$

$$G_b^{(j)} + v(t_k), \quad t_k \in T_j \tag{1}$$

$I(t_k)$ représentant une perfusion de médicament et $y(tk)^{(j)}$ étant un niveau de glucose à un instant d'échantillonnage $t_k$, $v(t_k) \sim N(0, \sigma_v)$ correspondant à une perturbation de bruit de processus avec une variance $\sigma_v^2$, et $T_j$ représentant les instances de temps dans un ensemble de données j, et les paramètres du modèle de réponse impulsionnelle finie $a = [a_0, a_1... a_n]$ ayant une dépendance au glucose ;
pour chaque niveau de glucose G d'intérêt, le problème suivant étant résolu pour retrouver les estimations des paramètres $a$ et $Gb = [G_b^{(1)}... G_b^{(N)}]$ en utilisant N périodes de données :

$$\{\mathbf{a}(G), \mathbf{G_b}\} = \arg\min \sum_{j=1}^{N} (\|\mathbf{y}^{(j)} - \hat{\mathbf{y}}^{(j)}\|_{W^G} + \alpha\|\mathbf{a}\|_R$$
$$+ \beta\|G_b^{(j)} - G_b^0\|_2) + \gamma\|\mathbf{a}\|_1 \tag{2}$$

sous réserve de

$$a_k \leq 0, , \quad k = [1, \ldots, n-1]$$
$$a_0 = 0$$
$$a_n = 0 \tag{3}$$

et une matrice de régularisation de second ordre R étant remplie comme suit :

$$R(j, j-1 : j+1) = \begin{bmatrix} 1 & -2 & 1 \end{bmatrix}, \quad j = [1, \ldots, n] \tag{4}$$

$\mathbf{y}^{(j)} = [y_{t_1^j} \cdots y_{t_n^j}]$ étant les données de glucose collectée pour l'enregistrement de données j couvrant les temps d'échantillonnage $\mathbf{T_j} = [t_1^j \cdots t_n^j]$, et $\hat{\mathbf{y}}^{(j)} = [\hat{y}_{t_1^j} \cdots \hat{y}_{t_n^j}]$ étant l'estimation correspondante du niveau de glucose selon l'équation (1) ; $W^G$ étant la matrice quadratique du noyau définissant le poids de chaque mesure de glucose en fonction de la distance au niveau de glucose $G$ ; $G_b^0$ étant l'estimation de la valeur attendue antérieure de $G_b$, et $\alpha, \beta$ et $\gamma$ étant des termes de pénalité.

2. Dispositif selon la revendication 1, configuré en outre pour estimer une dose requise nécessaire, pendant différents intervalles de temps, pour obtenir un effet escompté du médicament sur le glucose.

3. Dispositif selon l'une quelconque des revendications 1 ou 2, configuré en outre pour calculer le facteur de sensibilité du médicament pour différentes valeurs de glucose G en additionnant tous les termes *a(G) = [a$_0$, (G) a$_1$ (G)... an (G)]* donnés par le procédé selon la revendication 1.

4. Dispositif selon la revendication 3, configuré en outre pour calculer un facteur de sensibilité moyen du médicament en faisant la moyenne des facteurs de sensibilité selon la revendication 3.

5. Dispositif selon la revendication 4, configuré en outre pour calculer une dose de médicament sur la base du facteur de sensibilité, de la forme et de la durée de l'action du médicament, des informations sur les doses précédentes $D_j$ aux instants $T_j$, du niveau de glucose actuel G et du niveau de glucose cible Gt selon :

$$D = \max(0, \frac{G - G_t - \bar{K}_{ISR} \cdot D_{IOB}}{K_{ISR}}) \tag{8}$$

et

$$D_{IOB} = \sum_{j=1}^{m} D_j \left(1 - \frac{\sum_{i=1}^{(T-T_j)/5} \bar{a}_i}{K_{ISR}}\right), \quad \forall j : T_j \leq T - 5 \cdot n$$

$$(9)$$

6. Dispositif selon l'une quelconque des revendications précédentes, configuré pour utiliser les paramètres estimés selon la revendication 1 avec des informations de dosage planifié et des mesures du glucose, pour calculer un effet attendu pour une période de temps couvrant p pas d'échantillon à venir selon :

$$\hat{y}_{t_k|t_k} = (1 - \alpha)\hat{y}_{t_k|t_{k-1}} + \alpha y_t$$

$$(10)$$

$$\hat{y}_{t_{k+1}|t_k} = \hat{y}_{t_k|t_k} + \sum_{i=0}^{n} a_i(y(t_k))I(t_{k-i}) + \hat{G}_{b,T}$$

$$(11)$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \hat{G}_{b,T}, \quad 1 \leq j \leq p$$

$$(12)$$

$\hat{y}_{t_k}|t_k$ étant la mise à jour de la mesure, $\hat{y}_{t_k}|t_k$ étant la mise à jour temporelle, $\hat{y}_{t_{k+j+1}}|t_{k+j}$ étant la valeur prédite un pas en avant, et $\alpha$ étant la constante du filtre de Kalman, et $I(t_k)$ étant la dose d'insuline à l'instant $t_k$ ; et pour émettre au moins une alarme dans un dispositif électronique lorsque des seuils prédéfinis sont franchis.

7. Dispositif selon l'une quelconque des revendications précédentes, configuré pour utiliser les paramètres estimés selon la revendication 1, et des mesures du glucose pour simuler l'effet de différentes alternatives de dosage pour une période de temps couvrant p pas d'échantillon à venir selon :

$$\hat{y}_{t_k|t_k} = (1 - \alpha)\hat{y}_{t_k|t_{k-1}} + \alpha y_t$$

$$(10)$$

$$\hat{y}_{t_{k+1}|t_k} = \hat{y}_{t_k|t_k} + \sum_{i=0}^{n} a_i(y(t_k))I(t_{k-i}) + \hat{G}_{b,T}$$

$$(11)$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \hat{G}_{b,T}, \quad 1 \leq j \leq p$$

$$(12)$$

$\hat{y}_{t_k}|t_k$ étant la mise à jour de la mesure, $\hat{y}_{t_{k+1}}|t_k$ étant la mise à jour temporelle, $\hat{y}_{t_{k+j+1}}|t_{k+j}$ étant la valeur prédite un pas en avant, et $I(t_k)$ étant la dose d'insuline à l'instant $t_k$ et $\alpha$ étant la constante du filtre de Kalman.

8. Dispositif selon la revendication 1, configuré pour utiliser les paramètres estimés selon la revendication 1, et des mesures du glucose pour simuler l'effet de différentes alternatives de dosage pour une période de temps couvrant p pas d'échantillon à venir selon :

$$\hat{y}_{t_k|t_k} = (1 - \alpha)\hat{y}_{t_k|t_{k-1}} + \alpha y_t \tag{10}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{11}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$
$$+ \hat{G}_{b,T}, \quad 1 \le j \le p \tag{12}$$

$\hat{y}_{t_k}|_{t_k}$ étant la mise à jour de la mesure, $\hat{y}_{t_k}|_{t_k}$ étant la mise à jour temporelle, $\hat{y}_{t_{k+j+1}}|_{t_{k+j}}$ étant la valeur prédite un pas en avant, $I(t_k)$ étant la dose d'insuline à l'instant $t_k$, et $\alpha$ étant la constante du filtre de Kalman ; et pour optimiser une thérapie, $I_s = [I_T...I_{T+L}]$ étant la dose d'insuline suggérée au moment du calcul (numéro d'échantillon $T$) et $L(\le p)$ échantillons en avant selon :

$$\min_{I_s} C(\hat{Y} - Y_R) \tag{13}$$

sous réserve de

$$\hat{y}_j \ge \gamma_{\text{faible}} \quad = [T \dots T + L] \tag{14}$$

C étant une fonction de coût convexe, $\hat{Y} = [\hat{y}_T... \hat{y}_{T+L}]^T$ étant le tracé du glucose simulé calculé selon les équations (10)-(12), $Y_R = [y_R... y_R]$ étant un vecteur de même taille que $\hat{Y}$ de la valeur de glucose cible $y_R$.

9. Dispositif selon l'une quelconque des revendications précédentes, configuré pour utiliser les paramètres estimés selon la revendication 1, avec des informations sur la dose planifiée et des mesures du glucose, pour calculer un effet attendu et pour reprogrammer des doses planifiées dans une pompe d'administration de médicament lorsque des seuils prédéfinis sont franchis, la nouvelle dose de reprogrammation $I_s = [I_T...I_{T+L}]$ au moment du calcul (numéro d'échantillon $T$) et $L(\le p)$ échantillons à venir étant basée sur une optimisation selon :

$$\min_{I_s} C(\hat{Y} - Y_R) \tag{13}$$

sous réserve de

$$\hat{y}_j \ge \gamma_{\text{faible}} \quad j = [T \dots T + L] \tag{14}$$

C étant une fonction de coût convexe, $\hat{Y} = [\hat{y}_T...\hat{y}_{T+L}]^T$ étant le glucose simulé selon le tracé des Eq (10-12), $Y_R = [y_R... y_R]$ étant un vecteur de même taille que $\hat{Y}$ de la valeur de glucose cible $y_R$.

10. Dispositif selon l'une quelconque des revendications précédentes, configuré pour réaliser des calculs pour déterminer une estimation personnalisée des paramètres du modèle de réponse impulsionnelle finie $\mathbf{b^r} = [b^r_1, b^r_2 ...b^r_n]$

d'un effet d'élévation du glucose d'une prise de repas de la recette r, selon une approche de maximum de vraisemblance où la vraisemblance totale est maximisée :

$$\max_{\mathbf{b},\lambda} \quad p(Y, I, M_r, G_b, \lambda, b) \propto p(Y|G_b, \lambda, b) \cdot p(G_b, \lambda) \cdot p(b) \tag{15}$$

et $Y = [y_1^{(1)}\_\ldots y_n^{(1)}\_\ldots y_1^{(N)}\_y_n^{(N)}]$ étant la référence de glucose concaténée pour toutes les instances de repas, calculée selon :

$$y(t_k)^{(j)} = y(t_{k-1})^{(j)} + \lambda_j \sum_{i=0}^{n} a_i(y(t_k)^{(j)})I(t_{k-i})$$

$$+ \sum_{r=1}^{R} \sum_{i=0}^{m} b_i^r M_r^{(j)}(t_{k-i}) + G_b^{(j)} + v(t_k), \quad t_k \in T_j \tag{16}$$

$I(t_k)$ représentant la perfusion de médicament à l'instant $t_k$, $M_r(t_k)$ étant la prise de repas en grammes d'hydrates de carbone dans la recette r à l'instant d'échantillonnage $t_k$, $v(t_k) \sim N(0, \sigma_v)$ correspondant à une perturbation de bruit de processus, avec une variance $\sigma_v^2$, $T_j$ représentant les instances de temps dans l'ensemble de données j, et les paramètres du modèle de réponse impulsionnelle finie *a* étant tels que selon la revendication 1.

11. Dispositif selon l'une quelconque des revendications précédentes configuré pour utiliser les paramètres estimés selon la revendication 1 et 10 avec des informations de la dosage planifié et des mesures de glucose, pour calculer l'effet attendu pour une période de temps couvrant p pas d'échantillon à venir selon :

$$\hat{y}_{t_k|t_k} = (1 - \alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i(\hat{y}_{t_{k+j}|t_{k+j-1}})I(t_{k+j-i})$$

$$+ \sum_{r=1}^{R} \sum_{i=0}^{m} b_i^r M_r^{(j)}(t_{k-i}) + \hat{G}_{b,T}, \quad 1 \le j \le p \tag{20}$$

et pour émettre au moins une alarme dans un dispositif électronique lorsque des seuils prédéfinis sont franchis.

12. Dispositif selon l'une quelconque des revendications précédentes configuré pour utiliser les paramètres estimés selon les revendications 1 et 10, et des mesures de glucose pour simuler l'effet de différentes alternatives de dosage pour une période de temps couvrant p pas d'échantillon à venir selon :

$$\hat{y}_{t_k|t_k} = (1-\alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i\left(\hat{y}_{t_{k+j}|t_{k+j-1}}\right)I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p \tag{20}$$

**13.** Dispositif selon l'une quelconque des revendications précédentes configuré pour utiliser les paramètres estimés selon l'une quelconque des revendications 1 et 10, et des mesures récentes de glucose pour simuler l'effet de différentes alternatives de dosage pour une période de temps couvrant p pas d'échantillon à venir selon :

$$\hat{y}_{t_k|t_k} = (1-\alpha)\hat{y}_{t_k|t_{k-1}} + \alpha y_t \tag{18}$$

$$\hat{y}_{t_{k+1}|t_k} = \hat{y}_{t_k|t_k} + \sum_{i=0}^{n} a_i(y(t_k))I(t_{k-i}) + \hat{G}_{b,T} \tag{19}$$

$$\hat{y}_{t_{k+j+1}|t_{k+j}} = \hat{y}_{t_{k+j}|t_{k+j-1}} + \sum_{i=0}^{n} a_i\left(\hat{y}_{t_{k+j}|t_{k+j-1}}\right)I(t_{k+j-i})$$
$$+ \sum_{r=1}^{R}\sum_{i=0}^{m} b_i^r M_r^{(j)}(t_{k-i}) + \hat{G}_{b,T}, \quad 1 \le j \le p \tag{20}$$

et pour optimiser une thérapie $I_s = [I_T \ldots I_{T+L}]$ au moment du calcul (numéro d'échantillon $T$) et $L(\le p)$ échantillons à venir selon :

$$\min_{I_s} C(\hat{Y} - Y_R) \tag{13}$$

sous réserve de

$$\hat{y}_j \ge \gamma_{\text{faible}} \quad j = [T \ldots T+L] \tag{14}$$

C étant une fonction de coût convexe, $\hat{Y}=[\hat{y}_T...\hat{y}_{T+L}]^T$ étant le glucose simulé selon le tracé des Eq (10-12), $\mathbf{Y_R}=[y_R...y_R]$ étant un vecteur de même taille que $\hat{Y}$ de la valeur de glucose cible $y_R$.

14. Système comprenant le dispositif selon les revendications 1 à 13, et comprenant en outre une pompe à insuline, un dispositif de mesure de glucose, une unité de réception pour un dispositif de mesure de glucose en continu, un smartphone, une tablette, un ordinateur ou tout autre dispositif pouvant avoir la capacité d'afficher des informations à un utilisateur et de réaliser les mesures et les calculs nécessaires.

Fig. 1

Fig. 2

Fig. 3

Fig. 4

Fig. 5

**REFERENCES CITED IN THE DESCRIPTION**

**Patent documents cited in the description**

- US 2005272640 A